# FASCICULE DE BREVET EUROPEEN

(11) **EP 0 469 993 B1**
(45) Date de publication et mention de la délivrance du brevet: **31.05.1995**
(21) Numéro de dépôt: 91402147.2
(22) Date de dépôt: 31.07.1991
(51) Int. Cl.: A61F 2/14

(54) **Cornée artificielle**
Künstliche Hornhaut
Artificial cornea

(30) Priorité: 02.08.1990 FR 9009915
(43) Date de publication de la demande: 05.02.1992
(73) Titulaire: Lacombe, Emmanuel, F-75017 Paris (FR)
(72) Inventeur: Lacombe, Emmanuel, F-75017 Paris (FR)
(74) Mandataire: Dronne, Guy

(56) Documents cités:
- FR-A- 2 608 041
- GB-A- 1 044 420
- US-A- 4 470 159

## Description

La présente invention concerne une cornée artificielle.

De façon plus précise, l'invention se rapporte à une prothèse optique qui peut être mis en place par une trépanation centrale pratiquée dans la cornée lorsque celle-ci ne peut plus remplir sa fonction de transparence.

Certaines cécités peuvent être provoquées par une opacification ou altération des propriétés optiques de la cornée. Dans certains cas, il est possible d'y remédier par kératoplastie, c'est-à-dire en remplaçant la partie de la cornée malade par un fragment de cornée saine et transparente d'un donneur. Cependant, si les lésions dont est atteinte la cornée sont trop importantes (brûlure, syndrome sec, pseudo-pemphigus ...), il faut avoir recours à une chirurgie palliative qui consiste à ménager un orifice ou trépanation dans la partie malade de la cornée et à mettre en place une prothèse formant cornée artificielle, cette technique est dite de kératoprothèse.

Deux techniques de kératoprothèse sont déjà bien connues : la technique d'odonto-kératoprothèse selon Strampelli ou la technique de kératoprothèse selon Choyce. Ces techniques chirurgicales utilisent, soit une fixation antérieure du support de la prothèse sur la cornée, soit une fixation intra-cornéenne du support de prothèse.

Ces techniques présentent un certain nombre d'inconvénients (voir document US-A-4 470 159).

D'une part, elles font appel à des gestes chirurgicaux extrêmement lourds et provoquent des complications post-opératoires importantes qui aboutissent souvent à des résultats décevants pour le malade aussi bien du point de vue fonctionnel qu'anatomique. Les modes de fixation de la prothèse rappelés ci-dessus exposent le malade à des nécroses des plans de recouvrement qui peuvent entraîner l'expulsion de la prothèse.

D'autre part, lors de la mise en place des prothèses des types rappelés ci-dessus, on a souvent noté la formation sur la cornée d'un voile fibreux sur la face postérieure de la cornée et, ce qui est une cause d'échecs, sur la face postérieure de la prothèse rendant celle-ci au moins partiellement inopérante.

Un objet de l'invention est de fournir une prothèse formant cornée artificielle qui remédie aux inconvénients rappelés ci-dessus.

Pour atteindre ce but, selon l'invention, la cornée artificielle se caractérise en ce qu'elle comprend
une pièce optique, au moins partiellement transparente, de forme générale cylindrique dont la section droite est sensiblement identique à celle de l'orifice ménagé dans la cornée, présentant une première extrémité et une deuxième extrémité située dans la chambre antérieure de l'oeil lorsque ladite pièce optique est mise en place ;
une pièce support solidaire au moins de la deuxième extrémité de ladite pièce optique entourant ladite deuxième extrémité et présentant une face d'appui en regard de la face postérieure de la cornée lorsque ladite pièce support est mise en place, ladite face d'appui présentant des dimensions externes supérieures à celles de la section droite dudit orifice ; et
des moyens de fixation pour appliquer avec pression au moins temporairement ladite face d'appui de ladite pièce support contre la face postérieure de la cornée à la périphérie dudit orifice.

On comprend qu'ainsi la fixation définitive de la prothèse dans la cornée se fait notamment par l'adhérence de la paroi postérieure de la cornée sur la pièce support, ce qui permet de plus d'obtenir une liaison étanche entre la prothèse et la cornée, et qui permet d'éviter l'expulsion de la kératoprothèse. De préférence, ladite pièce support comporte en outre des moyens pour assurer une fixation et un positionnement au moins temporaire de ladite pièce support sur la face postérieure de la cornée.

De préférence encore lesdits moyens de fixation comprennent une pièce de fixation externe à l'oeil, solidaire de la première extrémité de la pièce optique et prenant appui sur la périphérie de la face avant de la cornée.

Selon un mode préféré de mise en oeuvre, ladite pièce optique est solidaire de ladite pièce support par un ensemble filetage-taraudage ménagé dans la deuxième extrémité de la pièce optique et dans ladite pièce support.

D'autres caractéristiques et avantages de l'invention apparaîtront plus clairement à la lecture de la description qui suit de plusieurs modes de réalisation de l'invention donnés à titre d'exemples non limitatifs. La description se réfère aux dessins annexés sur lesquels :
- la figure 1 est une vue partielle en coupe verticale d'un globe oculaire dans lequel une kératoprothèse selon l'invention a été mise en place ;
- la figure 2 est analogue à la figure 1 mais elle illustre le cas où l'épaisseur centrale de la cornée est plus importante ; et
- la figure 3 est une vue en coupe verticale des trois parties constituant la prothèse selon un mode préféré de réalisation de l'invention.

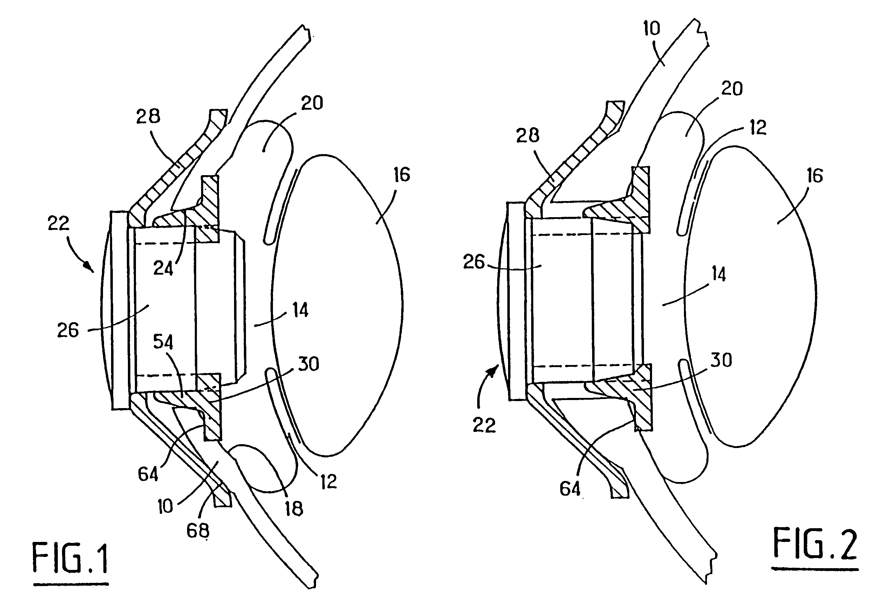

En se référant tout d'abord à la figure 1, on va décrire l'organisation générale de la kératoprothèse ainsi que la façon de la mettre en place dans l'oeil.

Sur la figure 1, on a représenté en coupe verticale la partie antérieure d'un globe oculaire. On a représenté en particulier la cornée 10, l'iris 12 avec la pupille 14, ainsi que le cristallin 16 situé bien sûr derrière l'iris 12 et la pupille 14. Entre l'iris 12 et la face postérieure 18 de la cornée 10, on trouve la chambre antérieure 20 de l'oeil. Comme on l'a déjà indiqué précédemment, la cornée artificielle qui porte la référence générale 22 est placée dans un orifice 24 ou trépanation qui a été ménagé dans la cornée 10. Comme le montre de façon simplifiée la figure 1, la prothèse 22 est constituée essentiellement par trois pièces. On trouve, tout d'abord, une pièce optique 26 réalisée en un matériau transparent et dont la fonction est de remplacer la partie malade de la cornée 10. La prothèse comporte une pièce support 30 qui, par sa partie périphérique, prend appui sur la paroi postérieure 18 de la cornée 10. Cette pièce support 30 est solidaire de l'extrémité postérieure de la pièce optique 26. Enfin, la prothèse peut comprendre une pièce de fixation 28 qui est disposée à l'extérieur de la cornée et qui, d'une part, est solidarisée avec l'extrémité avant de la pièce optique 26 et qui, d'autre part, prend appui sur la périphérie de la partie antérieure 10 de la cornée.

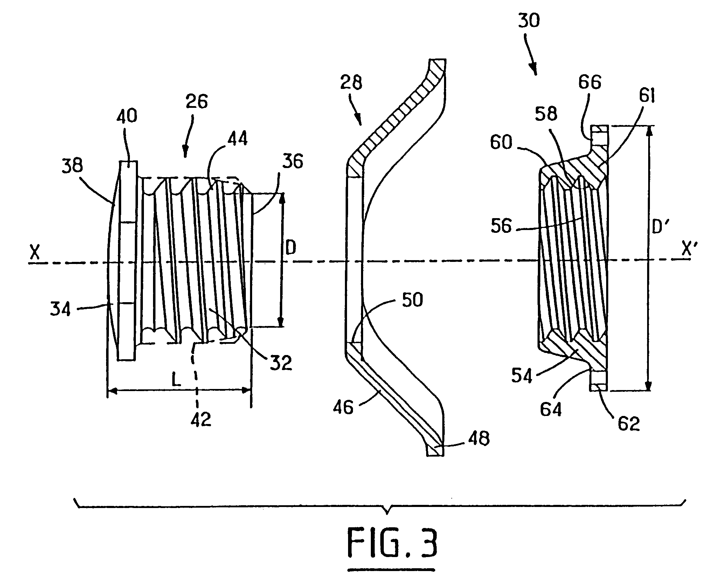

En se référant maintenant à la figure 3, on va décrire en détail le mode de réalisation préféré des trois pièces formant l'implant 22.

La pièce optique 26 est réalisée en un matériau transparent. Cette pièce 26 est par exemple réalisée en PPMA (polymétacrylate de méthyle) plus connu sous la marque Plexiglas. La pièce 26 a la forme générale d'un cylindre 32 d'axe X-X' et de section droite circulaire. Le cylindre 32 présente une première extrémité 34 ou extrémité antérieure et une deuxième extrémité 36 ou extrémité postérieure. L'extrémité antérieure 34 est définie par une surface convexe 38 qui est déterminée pour donner à la pièce 26 les paramètres optiques désirés. L'extrémité 34 comporte également une collerette 40 qui fait saillie radialement hors de la face latérale 42 du cylindre 32. Quant à la deuxième extrémité 36 de la pièce optique 26, elle est plane. Enfin, la face latérale 42 du cylindre 32 est munie d'un filetage 44.

Dans l'exemple considéré, la pièce optique 26 a une longueur totale L de l'ordre de 4 millimètres et sa partie cylindrique 32 a un diamètre D de l'ordre de 5 millimètres également. Les dimensions de la pièce optique 26 et la courbure 38 de sa face avant sont choisies pour produire un champ visuel correspondant à à peu près 70° et pour que le système optique ainsi constitué présente une vergence de l'ordre de 40 dioptries.

La pièce de fixation 28 a la forme générale d'une lame incurvée qui présente deux bords d'appui d'extrémité 48 à leurs extrémités évasées. Le centre de la lame 46 est percé d'un orifice axial 50 dont le diamètre est suffisant pour permettre le libre passage du cylindre 32 de la pièce optique 26 mais insuffisant pour laisser passer la collerette 40, de telle manière que celle-ci vienne en appui sur la face antérieure 52 de la partie centrale de la pièce de fixation 28.

Si l'on considère maintenant la pièce support 30, on voit que celle-ci a la forme générale d'un disque ou d'une couronne prolongé par un manchon 54 dont l'orifice axial 56 est muni d'un taraudage 58. Le taraudage 58 est déterminé pour coopérer avec le filetage 54 de la partie latérale de la pièce optique 26. Il faut veiller à ce que le vissage de la pièce optique 26 dans la pièce support 30 soit étanche au moins aux liquides. Comme le montre la figure 3, la paroi latérale du manchon 54, et qui est référencée 60, est légèrement conique. A son extrémité postérieure 61, le manchon 54 est muni d'une collerette 62 qui fait bien sûr saillie hors de la paroi latérale 60. La face antérieure 64 de la collerette 62 forme une face d'appui comme on l'expliquera ultérieurement. En outre, de préférence, la collerette 64 comporte des orifices 66 régulièrement répartis sur sa circonférence. Le diamètre D' de la collerette 62 est de l'ordre de 8 millimètres, c'est-à-dire sensiblement supérieur à celui de l'orifice 24 ménagé dans la cornée 10. Il faut ajouter que la pièce de fixation 28, comme la pièce support 30, sont de préférence réalisées également en PMMA.

Après avoir décrit en détail un mode préféré de réalisation de l'implant 10, on va décrire, en se référant plus particulièrement à la figure 1, le mode de mise en place de l'implant dans la cornée 10.

Dans un premier temps, de façon connue, on pratique la trépanation 24 dans le centre de la cornée. Ensuite, on met en place par les techniques habituellement utilisées pour les prothèses de chambre a ntérieure, la pièce de support 30 dans la chambre antérieure 20, de telle manière que la face d'appui 64 de la collerette 62 soit placée contre la face postérieure 18 de la cornée et que le manchon 54 de cette pièce soit au moins partiellement engagé dans l'orifice 24 de la cornée. Par exemple, la pièce support 30 est introduite dans la chambre antérieure par une incision pratiquée à la périphérie de la cornée. On réalise alors une fixation et un positionnement de la pièce 30 ainsi mise en place au moyen d'une suture à travers la cornée à l'aide des orifices 66 ménagés dans la pièce 30. En variante, la pièce support peut être entourée par un tissu poreux colonisable dans lequel la suture transcornéenne est réalisée.

Dans une étape ultérieure, on place la pièce de fixation 28 autour du cylindre 32 de la pièce optique 26 de telle manière que la face antérieure 52 de la pièce 28 soit en contact contre la collerette 40 de la pièce optique 26. L'extrémité postérieure 36 de la pièce optique 26 est alors engagée dans l'orifice 24 de la cornée de telle manière que celle-ci pénètre dans l'orifice taraudé 56 de la pièce support 30. On procède alors au vissage de la pièce 26 dans la pièce d'appui 30. Lors de ce vissage, le rebord périphérique 48 de la pièce de fixation 28, qui présente une certaine élasticité, vient en appui sur la sclérotique 68, ou la périphérie de la cornée 10, comme le montre la figure 1. Le vissage est interrompu lorsque, d'une part, la pièce optique 26 est placée correctement par rapport à la cornée et par rapport à la chambre antérieure 20, et que, d'autre part, la pièce de fixation 28 qui est partiellement élastique exerce un effort de traction convenable sur la pièce optique 26. Compte tenu de l'élasticité de la pièce 30, la face d'appui 64 de la pièce 30 est fermement appliquée contre la paroi postérieure 18 de la cornée. En outre, la pression exercée par l'humeur aqueuse sur face postérieure des pièces 26 et 30 favorise ce phénomène. Lorsque sa mise en place est réalisée, la pièce optique 26 peut affleurer la face postérieure de la pièce 30 ou dépasser de celle-ci d'environ un millimètre. Cette dernière disposition présente l'avantage d'éviter la constitution d'un voile sur la face postérieure de la pièce optique, du fait que celle-ci pénètre dans la chambre antérieure 20. La mise en place de la prothèse est alors terminée.

Il se forme progressivement, par cicatrisation, une adhérence entre la collerette 64 de la pièce support 30 et la paroi postérieure 18 de la cornée. Cette adhérence présente de plus l'avantage de constituer une liaison étanche entre ces deux pièces. Cette liaison est encore renforcée du fait de la formation, le plus souvent, d'un voile fibreux sur la face postérieure de la pièce support 30.

Lorsque la cicatrisation est totalement réalisée et que l'on a obtenu ainsi une liaison définitive et étanche entre la pièce 30 et la face postérieure de la cornée, il est possible de procéder à l'ablation de la pièce de fixation 28. Ainsi, seules demeurent de la prothèse la pièce de fixation 30 et la pièce optique 26. Il va de soi que, dans certains cas, on pourra juger préférable de laisser en place la pièce de fixation 28.

La figure 3 montre un autre avantage de l'invention. En effet, grâce à la liaison filetée entre la pièce support 30 et la pièce optique 26, il est possible d'adapter la prothèse à différentes épaisseurs centrales de la cornée en vissant plus ou moins la pièce optique 26 dans la pièce de fixation 30. Dans le cas de la figure 1, l'épaisseur centrale de la cornée est de l'ordre de 0,8 millimètre, alors que dans le cas de la figure 2, cette épaisseur est de l'ordre de 3 millimètres.

Un avantage supplémentaire réside dans le fait que, dans le mode de réalisation précédemment décrit, la pièce appliquée est vissée dans la pièce support. En cours de vie de la kératoprothèse, il est donc possible de changer la pièce optique 26 pour l'adapter aux changements de vision du porteur de la prothèse.

On pourrait donc envisager d'utiliser la kératoprothèse comme support d'un système optique correcteur, même dans le cas où la cornée est saine.

Dans la description précédente, on a décrit un mode préféré de réalisation de la prothèse selon l'invention. Il va cependant de soi que la prothèse peut présenter des variantes de réalisation. En particulier, la pièce support 30 et la pièce optique 26 pourraient être réalisées à l'aide d'une seule et même pièce qui serait alors globalement placée dans la cornée, de telle manière qu'on retrouve la configuration représentée sur les figures 1 et 2. De même, la pièce de fixation 28 peut présenter d'autres formes. Il suffit qu'elle comporte des moyens de solidarisation avec l'extrémité antérieure 34, 40 de la pièce optique 26 et des deuxièmes extrémités prenant appui sur la périphérie de la cornée pour assurer l'effort de traction nécessaire sur la pièce de fixation 30 par l'intermédiaire de la pièce optique 26. En outre, il est souhaitable de donner à la pièce de fixation 28 une forme convenable, par exemple des pattes flexibles solidaires d'une couronne, pour permettre aisément son ablation lorsque la pièce 30 adhère convenablement à la face postérieure de la cornée.

Selon le mode préféré de réalisation, la kératoprothèse comprend la pièce de fixation 28. Cependant, on ne sortirait pas de l'invention si la prothèse ne comportait pas cette pièce. En effet, dans certains cas, la suture transcornéenne de la pièce support, ajoutée à la pression exercée sur la face postérieure de cette pièce par l'humeur aqueuse remplissant la chambre antérieure, peut suffire pour plaquer la pièce 30 sur la face postérieure de la cornée.

## Revendications

1. Cornée artificielle destinée à être mise en place dans un orifice ménagé dans la partie centrale de la cornée, comprenant : une pièce support (30) munie d'une pièce d'appui présentant des dimensions externes supérieures à celles de la section droite dudit orifice (24), des moyens de fixation (28) de la piece optique et
une pièce optique (26), au moins partiellement transparente, de forme générale cylindrique dont la section droite est sensiblement identique à celle de l'orifice (24) ménagé dans la cornée (40), présentant une première extrémité (34) et une deuxième extrémité (36) caractérisée en ce que ladite deuxième extrémité est située dans la chambre antérieure de l'oeil lorsque ladite pièce optique est mise en place ;
que ladite pièce support (30) est solidaire au moins de la deuxième extrémité (36) de ladite pièce optique, entoure ladite deuxième extrémité et présente une face d'appui (64) en regard de la face postérieure de la cornée lorsque ladite pièce support est mise en place,
et en ce que lesdits moyens de fixation (28) sont prévus pour appliquer avec pression au moins temporairement ladite face d'appui de ladite pièce support contre la face postérieure de la cornée à la périphérie dudit orifice.

2. Cornée artificielle selon la revendication 1, caractérisée en ce que lesdits moyens de fixation comprennent une pièce de fixation (28), externe à l'oeil, solidaire de la première extrémité de la pièce optique (26) et prenant appui par sa périphérie sur la périphérie de la face avant de la cornée.

3. Cornée artificielle selon l'une quelconque des revendications 1 et 2, caractérisée en ce que ladite pièce support (30) comporte en outre des moyens (66) pour assurer une fixation et un positionnement au moins temporaire de ladite pièce support sur la face postérieure de la cornée.

4. Cornée artificielle selon l'une quelconque des revendications 1 à 3, caractérisée en ce que ladite pièce optique (26) est solidaire de ladite pièce support (30) par un ensemble filetage-taraudage (44, 58) ménagé dans la deuxième extrémité (36) de la pièce optique et dans ladite pièce support.

5. Cornée artificielle selon la revendication 2, caractérisée en ce que ladite pièce de fixation (28) comporte des moyens de coopération (52) avec la première extrémité (40) de ladite pièce optique, et des moyens périphériques d'appui (48) sur la périphérie de la partie antérieure de la cornée pour exercer une traction sur la pièce optique et donc sur la pièce support.

6. Cornée artificielle selon la revendication 3, caractérisée en ce que les moyens de fixation comprennent des orifices (64) percés à la périphérie de ladite pièce support pour réaliser une suture transcornéenne de celle-ci sur la face postérieure de la cornée.

7. Cornée artificielle selon l'une quelconque des revendications 1 à 6, caractérisée en ce que ladite pièce optique (26) est réalisée entièrement en un matériau transparent,
en ce qu'elle a la forme générale d'un cylindre (32) à section droite circulaire,
en ce que sa première extrémité (34) comporte une collerette (40) débordant dudit cylindre, et
en ce qu'au moins sa deuxième extrémité est filetée (44).

8. Cornée artificielle selon la revendication 7, caractérisée en ce que ladite pièce support (30) a la forme générale d'un disque circulaire présentant deux faces terminales, dont l'orifice central est taraudé (58) pour coopérer avec le filetage (44) de ladite pièce optique (26), et dont la périphérie de la face terminale tournée vers la face postérieure de la cornée, constitue ladite face d'appui (64).

9. Cornée artificielle selon la revendication 8, caractérisée en ce que ladite pièce de fixation (28) a la forme générale d'une lame élastique incurvée présentant un orifice central (50) pour coopérer avec la collerette (40) de ladite pièce optique et des extrémités périphériques (48) pour venir en appui sur la périphérie de la partie antérieure de la cornée.

10. Cornée artificielle selon la revendication 1, caractérisée en ce que ladite pièce optique (26) et ladite pièce support (30) ne forment qu'une seule et même pièce.

11. Cornée artificielle selon l'une quelconque des revendications 1 à 10, caractérisée en ce que lesdites trois pièces sont réalisées en PMMA.

12. Cornée artificielle selon l'une quelconque des revendications 7 à 9, 10, caractérisée en ce que la longueur du cylindre (32) formant ladite pièce optique (28) est de l'ordre de 4 millimètres et son diamètre est de l'ordre de 5 millimètres.

13. Cornée artificielle selon l'une quelconque des revendications 1 à 12, caractérisée en ce que ladite pièce optique (26) présente une vergence de l'ordre de 40 dioptries.

14. Cornée artificielle selon la revendication 2, caractérisée en ce que ladite pièce de fixation (28) est réalisée de telle manière qu'on puisse en faire l'ablation lorsque ladite pièce support (30) a adhéré à la paroi postérieure de la cornée.

15. Cornée artificielle selon l'une quelconque des revendications 1 à 14, caractérisée en ce qu'elle comporte un jeu de pièces optiques (26) présentant chacune des caractéristiques de correction visuelle différentes.

16. Cornée artificielle selon l'une quelconque des revendications 1 à 15, caractérisée en ce que ladite pièce support (30) est entourée par un tissu poreux colonisable.

## Claims

1. Artificial cornea for installing in an orifice formed in the central portion of the cornea, comprising a support part (30) provided with a bearing part the outside dimensions of which are greater than the outside dimensions of the right cross-section of said orifice (24), fixing means (28) for the optical part and an optical part (26) which is transparent, at least in part, which is generally in the form of a cylinder whose right cross-section is substantially identical to that of the orifice (24) formed in the cornea (40), which has a first end (34), and which has a second end (36), characterized in that sad second end is situated inside the anterior chamber of the eye when said optical part has been put into place;
in that said support part (30) is fixed at least to the second end (36) of said optical part, surrounds said second end, and has a bearing face (64) facing the posterior face of the cornea when said support part has been put into place;
and in that said fixing means (28) are provided for at least temporarily pressing said bearing face of said support part against the posterior face of the cornea at the periphery of said orifice.

2. Artificial cornea according to claim 1, characterized in that said fixing means comprise a fixing part (28) outside the eye, fixed to the first end of the optical part (26), and having its periphery bearing against the periphery of the front face of the cornea.

3. Artificial cornea according to any one of claims 1 and 2, characterized in that said support part (30) further includes means (66) for at least temporarily positioning and fixing said support part on the posterior face of the cornea.

4. Artificial cornea according to any one of claims 1 to 3, characterized in that said optical part (26) is fixed to said support part (30) by a co-operating screw thread and tapping (44, 58) formed at the second end (36) of the optical part and in said support part.

5. Artificial cornea according to claim 2, characterized in that said fixing part (28) includes means (52) for co-operating with the first end (40) of said optical part, and peripheral bearing means (48) for bearing against the periphery of the anterior portion of the cornea to exert traction on the optical part, and thus on the support part.

6. Artificial cornea according to claim 3, characterized in that the fixing means include orifices (64) pierced through the periphery of said support part to enable it to be attached to the posterior face of the cornea by means of a transcorneal suture.

7. Artificial cornea according to any one of claims 1 to 6, characterized in that said optical part (26) is made entirely of transparent material,
in that it is generally in the form of a right cross-section circular cylinder (32),
in that it has a flange (40) projecting outwards from the first end (34) of said cylinder, and
in that it is threaded (44) at least at its second end.

8. Artificial cornea according to claim 7, characterized in that said support part (30) is generally in the form of a circular disk having two end faces, with its central orifice being tapped (58) to co-operate with the thread (44) of said optical part (26), and with the periphery of its end face directed towards the posterior face of the cornea constituting said bearing face (64).

9. Artificial cornea according to claim 8, characterized in that said fixing part (28) is generally in the form of a curved resilient plate having a central orifice (50) for co-operating with the flange (40) of said optical part, and peripheral ends (48) for bearing against the periphery of the anterior portion of the cornea.

10. Artificial cornea according to claim 1, characterized in that said optical part (26) and said support part (30) are constituted by a single part.

11. Artificial cornea according to any one of claims 1 to 10, characterized in that all three parts are made of PMMA.

12. Artificial cornea according to any one of claims 7 to 9, 10, characterized in that the length of the cylinder (32) constituting said optical part (28) is about 4 millimeters and its diameter is about 5 millimeters.

13. Artificial cornea according to any one of claims 1 to 12, characterized in that said optical part (26) has a vergency of about 40 diopters.

14. Artificial cornea according to claim 2, characterized in that said fixing part (28) is made in such a manner as to enable it to be removed once said support part (30) adheres to the posterior wall of the cornea.

15. Artificial cornea according to any one of claims 1 to 14, characterized in that it includes a set of optical parts (26) each having different vision-correcting characteristics

16. Artificial cornea according to any one of claims 1 to 15, characterized in that said support part (30) is surrounded by porous fabric suitable for colonization.

## Patentansprüche

1. Künstliche Hornhaut, welche zum Einsetzen in eine im zentralen Teil der Hornhaut vorgesehene Öffnung bestimmt ist, mit einem Trägerstück (30), das mit einem Auflagestück mit größeren Außenabmessungen als jenen des Querschnitts der Öffnung (24) versehen ist, mit Befestigungsmitteln (28) eines optischen Stücks und mit einem optischen Stück (26), das zumindest teilweise transparent ist und eine allgemein zylindrische Form aufweist, deren Querschnitt im wesentlichen identisch ist mit jenem der in der Hornhaut (40) vorgesehenen Öffnung (24), und das ein erstes Ende (34) und ein zweites Ende (36) aufweist, dadurch gekennzeichnet, daß sich das zweite Ende in der vorderen Augenkammer befindet, wenn das optische Stück eingesetzt ist; daß das Trägerstück (30) zumindest mit dem zweiten Ende (36) des optischen Stücks fest verbunden ist, das zweite Ende umgibt, und eine Anlagefläche (64) gegenüber der hinteren Fläche der Hornhaut aufweist, wenn das Trägerstück eingesetzt ist; und daß die Befestigungsmittel (28) vorgesehen sind, um zumindest temporär die Anlagefläche des Trägerstücks mit Druck gegen die hintere Fläche der Hornhaut am Umfang der Öffnung anzulegen.

2. Künstliche Hornhaut nach Anspruch 1, dadurch gekennzeichnet, daß die Befestigungsmittel ein Befestigungsstück (28) umfassen, das außerhalb des Auges mit dem ersten Ende des optischen Stücks (26) fest verbunden ist und über seinen Umfang auf dem Umfang der vorderen Fläche der Hornhaut anliegt.

3. Künstliche Hornhaut nach einem der Ansprüche 1 oder 2, dadurch gekennzeichnet, daß das Trägerstück (30) weiters Mittel (66) zum Sicherstellen einer zumindest temporären Befestigung und Positionierung des Trägerstücks an der hinteren Fläche der Hornhaut umfaßt.

4. Künstliche Hornhaut nach einem der Ansprüche 1 bis 3, dadurch gekennzeichnet, daß das optische Stück (26) mit dem Trägerstück (30) durch eine im zweiten Ende (36) des optischen Stücks und im Trägerstück liegende Außen-Innen-Gewindeanordnung (44, 50) fest verbunden ist.

5. Künstliche Hornhaut nach Anspruch 2, dadurch gekennzeichnet, daß das Befestigungsstück (28) Mittel (52) zum Zusammenwirken mit dem ersten Ende (40) des optischen Stücks und periphere Mittel (48) zur Anlage am Umfang des vorderen Teils der Hornhaut umfaßt, um eine Zugkraft auf das optische Stück und daher das Trägerstück auszuüben.

6. Künstliche Hornhaut nach Anspruch 3, dadurch gekennzeichnet, daß die Befestigungsmittel Öffnungen (64) umfassen, die in den Umfang des Trägerstücks gebohrt sind, um ein transkorneales Annähen desselben an der hinteren Fläche der Hornhaut zu ermöglichen.

7. Künstliche Hornhaut nach einem der Ansprüche 1 bis 6, dadurch gekennzeichnet, daß das optische Stück (26) zur Gänze aus einem transparenten Material besteht, daß es allgemein die Form eines Zylinders (32) mit kreisförmigem Querschnitt hat, daß sein erstes Ende (34) einen über den Zylinder vorstehenden Bund (40) umfaßt, und daß zumindest sein zweites Ende mit einem Außengewinde (44) versehen ist.

8. Künstliche Kornhaut nach Anspruch 7, dadurch gekennzeichnet, daß das Trägerstück (30) allgemein die Form einer kreisförmigen Scheibe mit zwei Stirnflächen hat, wobei die zentrale Öffnung mit einem Innengewinde (58) versehen ist, um mit dem Außengewinde (44) des optischen Stücks (26) zusammenzuwirken, und wobei der Umfang der Stirnfläche, die der hinteren Fläche der Hornhaut zugewandt ist, die Anlagefläche (64) bildet.

9. Künstliche Hornhaut nach Anspruch 8, dadurch gekennzeichnet, daß das Befestigungsstück (28) allgemein die Form eines gekrümmten elastischen Blatts aufweist, mit einer zentralen Öffnung zum Zusammenwirken mit dem Bund (40) des optischen Stücks (50), und mit peripheren Enden (48) zur Anlage am Umfang des vorderen Teils der Hornhaut.

10. Künstliche Hornhaut nach Anspruch 1, dadurch gekennzeichnet, daß das optische Stück (26) und das Trägerstück (30) ausschließlich ein und dasselbe Stück bilden.

11. Künstliche Hornhaut nach einem der Ansprüche 1 bis 10, dadurch gekennzeichnet, daß die drei Stücke aus PMMA bestehen.

12. Künstliche Hornhaut nach einem der Ansprüche 7 bis 9 oder 10, dadurch gekennzeichnet, daß die Länge des das optische Stück (28) bildenden Zylinders (32) in der Größenordnung von 4 mm und sein Durchmesser in der Größenordnung von 5 mm liegt.

13. Künstliche Hornhaut nach einem der Ansprüche 1 bis 12, dadurch gekennzeichnet, daß das optische Stück (26) eine Brechkraft in der Größenordnung von 40 Dioptrien aufweist.

14. Künstliche Hornhaut nach Anspruch 2, dadurch gekennzeichnet, daß das Befestigungsstück (28) derart ausgebildet ist, daß es abgetragen werden kann, wenn das Trägerstück (30) an der hinteren Hornhautwand haftet.

15. Künstliche Hornhaut nach einem der Ansprüche 1 bis 14, dadurch gekennzeichnet, daß sie einen Satz optischer Stücke (26) mit jeweils unterschiedlichen Charakteristika zur Korrektur des Sehvermögens umfaßt.

16. Künstliche Hornhaut nach einem der Ansprüche 1 bis 15, dadurch gekennzeichnet, daß das Trägerstück (30) von porösem besetzbaren Gewebe umgeben ist.
